# EUROPÄISCHE PATENTSCHRIFT

(11) **EP 0 311 848 B1**
(45) Veröffentlichungstag und Bekanntmachung des Hinweises auf die Patenterteilung: **03.02.1993**
(21) Anmeldenummer: 88116103.8
(22) Anmeldetag: 29.09.1988
(51) Int. Cl.: A61M 1/16, A61M 1/34

(54) **Hämodialysegerät mit Schutzsystem gegen falsche Zusammensetzung der Dialysierflüssigkeit**
Hemodialysis with a protection system against incorrect composition of dialysis fluid
Appareil d'hémodialyse avec système de protection contre une concentration incorrecte du liquide de dialyse

(30) Priorität: 15.10.1987 DE 3734880
(43) Veröffentlichungstag der Anmeldung: 19.04.1989
(73) Patentinhaber: Fresenius AG, 61350 Bad Homburg (DE)
(72) Erfinder: Polaschegg, Hans-Dietrich, Dr., D-6370 Oberursel 4 (DE)
(74) Vertreter: Fuchs Mehler Weiss

(56) Entgegenhaltungen:
- EP-A- 0 160 272
- DE-A- 3 443 911

## Beschreibung

Die Erfindung bezieht sich auf eine Vorrichtung für die Hämodialyse mit einer Mischeinrichtung, die Dialysierflüssigkeit für einen Dialysator durch Mischung mindestens eines über eine Ansaugleitung aus einem Behälter zugeführten Dialysekonzentrates mit aufbereitetem Wasser herstellt, und mit einem vom Mischsystem unabhängigen, auf der Überwachung der Leitfähigkeit der Dialysierflüssigkeit beruhenden Schutzsystem, das verhindert, daß Dialysierflüssigkeit mit einer für den Patienten gefährlicher Zusammensetzung zum Dialysator gelangt, wie beispielsweise aus der EP-A 160 272 bekannt.

Hämodialysegeräte besitzen üblicherweise eine Misch-Einrichtung, die Dialysierflüssigkeit durch Mischung mindestens eines Dialysekonzentrates mit aufbereitetem Wasser für den Dialysator herstellt. Die Misch-Einrichtung besitzt dabei Ansaugstecker für die Ansaugleitungen der Konzentratbehälter.

Da solche Mischeinrichtungen versagen können, wird durch die gültigen Sicherheitsnormen ein vom Mischsystem unabhängiges Schutzsystem gefordert, das verhindert, daß Dialysierflüssigkeit mit für den Patienten gefährlichen Zusammensetzung zum Dialysator gelangt. Die Kontrolle der Leitfähigkeit der Dialysierflüssigkeit gilt dabei als hinreichendes Schutzsystem (VDE 750/Teil 206).

Es ist beispielsweise aus der eingangs erwähnten EP-A 160 272 bekannt, ein volumetrisches Mischsystem mit einem unabhängigen Schutzsystem auf der Basis der Leitfähigkeitsüberwachung der Dialysierflüssigkeit zu kombinieren. Dieses System bietet hinreichend Schutz vor der Dialyse mit falscher Dialysierflüssigkeitszusammensetzung, auch für den Fall eines beliebigen ersten Fehlers.

Aus medizinischen Gründen hat in den letzten Jahren die Hamodialyse mit bicarbonathaltiger Dialysierflüssigkeit Verbreitung gefunden. Aus Gründen der Stabilität muß in diesem Fall die Dialysierflüssigkeit durch Mischung von Wasser mit zwei Konzentraten hergestellt werden.

Neben den Fällen, daß zwei verschiedene Konzentrate mit unterschiedlichen Mischungsverhältnissen mit Wasser gemischt werden sollen, gibt es auch Misch-Einrichtungen, bei denen nur bestimmtes Konzentrat mit vorgegebenem Mischverhältnis eingemischt wird.

In der Regel werden die Konzentrate in Behältern (Kanistern) bereitgestellt. Da nun mehrere unterschiedliche Konzentrate in einer Dialysestation vorhanden sind, kann durch Verwechslung von Konzentrat-Behältern eine zusätzliche Gefährdung auftreten. Es wurde gezeigt, daß es Möglichkeiten der Verwechslung gibt, die zu einer für den Patienten gefährlichen Zusammensetzung der Dialysierflüssigkeit führen, die durch das Leitfähigkeits-Schutzsystem nicht erkannt werden können.

Zur Vermeidung solcher Gefährdungen ist es bekannt, zum Anschluß der Konzentratbehälter an die Ansaugstecker "kodierte Stecker" vorzusehen, die einen unverwechselbaren Anschluß des gewünschten Konzentratbehälters an den zugehörigen Ansaugstecker sicherstellen. Das System der kodierten Stecker setzt jedoch voraus, daß die Mischeinrichtung bzw. das Gerät und die Konzentratbehäter von demselben Hersteller stammen, da insoweit noch keine Normierung vorgenommen worden ist. Die Sicherheit durch kodierte Stecker ist daher nicht mehr gegeben, wenn Konzentratbehälter anderer Bauart vorhanden sind.

Es ist ferner bekannt, ein Schutzsystem auf pH-Elektrodenbasis vorzusehen.

Ein solches Schutzsystem hat jedoch nur bedingten Wert, da seine Funktion unter den üblichen Bedingungen des Hämodialysebetriebes nicht wie die übrigen Schutzsysteme vor Beginn einer Hämodialysebehandlung auf Funktion prüfbar ist. Dazu kommt, daß die Lebensdauer von pH-Elektroden unter den Bedingungen der Hämodialyse in der Regel kleiner als ein Jahr sind.

Der Erfindung liegt die Aufgabe zugrunde, ausgehend von den eingangs bezeichneten Vorrichtungen für die Hämodialyse mit einer Mischeinrichtung, die Dialysierflüssigkeit für einen Dialysator durch Mischung mindestens eines, über eine Ansaugleitung aus einem Behälter zugeführten Dialysekonzentrates mit aufbereitetem Wasser herstellt, und mit einem vom Mischsystem unabhängigen, auf der Überwachung der Leitfähigkeit der Dialysierflüssigkeit beruhenden Schutzsystem, das verhindert, daß Dialysierflüssigkeit mit einer für den Patienten gefährlichen Zusammensetzung zum Dialysator gelangt, diese so auszubilden, daß ein wirksamer Schutz gegen die mit falscher Zusammensetzung der Dialysierflüssigkeit gegen die Verwendung eines falschen Dialysekonzentrats erzielt werden kann.

Die Lösung dieser Aufgabe gelingt gemäß der Erfindung dadurch, daß in der Ansaugleitung eine zusätzliche Leitfähigkeitszelle angeordnet ist, deren Ausgangssignal auf eine Überwachungsstufe des Schutzsystems geschaltet ist, an der die Soll-Leitfähigkeit bei richtigem Anschluß des Dialyse-Konzentratbehälters voreingestellt ist und die ein Alarm- oder Steuersignal abgibt, wenn die gemessene Leitfähigkeit aufgrund eines falschen Anschlusses des Dialyse-Konzentratbehälters von dem voreingestellten Wert abweicht.

Da jedes Konzentrat eine spezifische Leitfähigkeit hat, d.h. die Leitfähigkeit das Konzentrat "identifiziert", ist sichergestellt, daß der Anschluß eines falschen Konzentratbehälters sofort erkannt wird.

Gleiches gilt, wenn zwei Konzentratbehälter mit unterschiedlichen Konzentraten vertauscht angeschlossen wurden.

Die Erfindung schafft daher ein wirksames Schutzsystem gegen Dialyse mit falscher Zusammensetzung der Dialysierflüssigkeit aufgrund von falsch angeschlossenen Konzentratbehältern.

Anhand von in den Zeichnungen dargestellten Ausführungsbeispielen wird die Erfindung näher erläutert.

Es zeigen :
1. Fig. 1 eine schematische Darstellung eines Hämodialysegerätes, bei der die Erfindung Anwendung findet,
2. Fig. 2 eine schematische Darstellung eines der Konzentratbehälter der Figur 1.
3. Fig.3 eine teilweise geschnittene Darstellung einer kompletten Ausgangsrohrleitung.

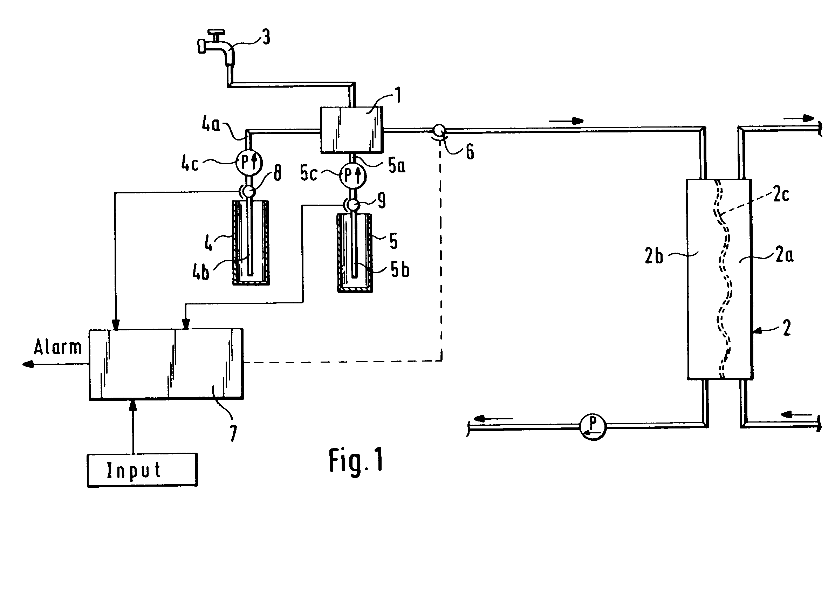

In Fig. 1 ist eine Ausführungsform für eine Vorrichtung für die Hämodialyse nach der Erfindung dargestellt. Die Vorrichtung weist eine Mischeinrichtung 1 auf, die Dialysierflüssigkeit für einen Dialysator 2 herstellt. Dieser Dialysator 2 besteht in bekannter Weise aus einem Blutteil 2a und einem Dialysierflüssigkeitsteil 2b, die durch eine Membran 2c abgetrennt sind.

Die Dialysierflüssigkeit wird in bekannter Weise durch Mischung von zwei Dialysekonzentraten mit aufbereitetem Wasser in der Mischeinrichtung 1 hergestellt. Das aufbereitete Wasser wird bei 3 entnommen. Die Konzentrate sind in Behältern 4 und 5 untergebracht, in die Ansaugrohre 4b, 5b eintauchen und die über Ansaug-Schläuche 4a und 5a mit den zugeordneten Konzentratansaugsteckern an der Mischeinrichtung 1 verbunden sind.

Die Konzentrate werden aus den Behältern 4 und 5 jeweils über Konzentratpumpen 4c und 5c in die Mischeinrichtung gefördert. Die Konzentratpumpen 4c, 5c sowie die Frischwasserzuführungseinrichtung werden von einer nicht gezeigten Steuereinrichtung gesteuert, was aus dem Stand der Technik bekannt ist.

In einem Konzentratbehälter soll sich beispielsweise das sogenannte "Säurekonzentrat", eine ca. 4-molare Säurekonzentratlösung, in dem anderen Behälter ein Bicarbonatkonzentrat (1-molare Lösung) befinden. Um die richtige Zusammensetzung der Dialysierflüssigkeit zu erhalten, muß das erste Konzentrat im Verhältnis 1 : 35, das Bicarbonatkonzentrat im Verhältnis 1 : 26 zur fertigen Lösung zugemischt werden.

Da auf einer Dialysestation unterschiedliche Konzentrate vorhanden sind, kann durch eine Verwechslung von Konzentraten insoweit eine zusätzliche Gefährdung auftreten.

Mit 6 ist ein Leitfähigkeitsmeßgerät eines vom Mischsystem unabhängigen Schutzsystems bezeichnet, dessen Ausgangssignal auf eine Überwachungsschaltung 7 geschaltet ist. Das Schutzsystem verhindert in bekannter Weise, daß Dialysierflüssigkeit mit für den Patienten gefährlicher Zusammensetzung zum Dialysator 2 gelangt.

Es wurde jedoch angezeigt, daß es Möglichkeiten der Verwechslung von Konzentratbehältern gibt, die zu einer für den Patienten gefährlichen Zusammensetzung der Dialysierflüssigkeit führen, die durch das Leitfähigkeitsschutzsystem mit der Zelle 6 nicht erkannt werden können.

Um diese zusätzliche Gefährdung wirksam auszuschließen, ist in dem Ansaugteil 4a, 4b; 5a, 5b jeweils eine Leitfähigkeitszelle 8, 9 angeordnet, deren Ausgangssignal auf die Überwachungsstufe 7 geschaltet ist. Die Leitfähigkeit der 1-molaren Bicarbonatlösung und der ca. 4-molaren Säurekonzentratlösung unterscheiden sich voneinander so stark, daß selbst nach Berücksichtigung aller einzubeziehenden Toleranzen ein deutlicher Leitfähigkeitsunterschied bleibt. So kann z.B. auch auf eine Temperaturkompensation verzichtet werden, da die dadurch hervorgerufenen Abweichungen nur in der Größenordnung von etwa 20 - 30 % liegen.

Wertet man statt der Absolutleitfähigkeit des einzelnen Konzentrates deren Verhältnis aus, so sinkt der Einfluß unterschiedlicher Temperaturen auf wenige % unter der Annahme, daß beide Konzentrate dieselbe Temperatur haben, was in der Regel gegeben ist.

Durch Vergleich der von den Leitfähigkeitszellen abgegebenen Ausgangssignale mit den voreingestellten Soll-Leitfähigkeitswerten können daher Konzentratverwechslungen sicher erkannt werden, insbesondere jene, die zu einer Dialysierflüssigkeit mit einer Leitfähigkeit im Erwartungsbereich führen würden, d.h. die das übergeordnete Schutzsystem mit der Meßzelle 6 nicht ansprechen lassen würden, weil die gemessene Leitfähigkeit der Dialysierflüssigkeit im Sollbereich liegt.

Die auftretenden Signal-Abweichungen in der Überwachungsstufe können einen Schalt- bzw. Steuervorgang in der Mischeinrichtung und/oder ein Alarmsignal auslösen.

Das Ziel einer einfachen und sicheren Schutzanordnung gegen Konzentratverwechslungen ist somit erreicht.

Die Erfindung ist nicht auf das angegebene Konzentratbeispiel beschränkt. Sie kann Anwendung finden bei allen Konzentraten, die eine deutlich unterschiedliche Leitfähigkeit zueinander aufweisen.

Es ist auch möglich, mit dem erfindungsgemäßen Prinzip solche Mischeinrichtungen zusätzlich zu überwachen, die nur einen Konzentratansaugstecker haben. Der jeweils gemessene Leitfähigkeitswert des angeschlossenen Konzentrates wird dann mit dem in der Überwachungsstufe eingestellten Sollwert des Leitfähigkeitswertes des tatsächlich auszuschließenden Konzentrates verglichen.

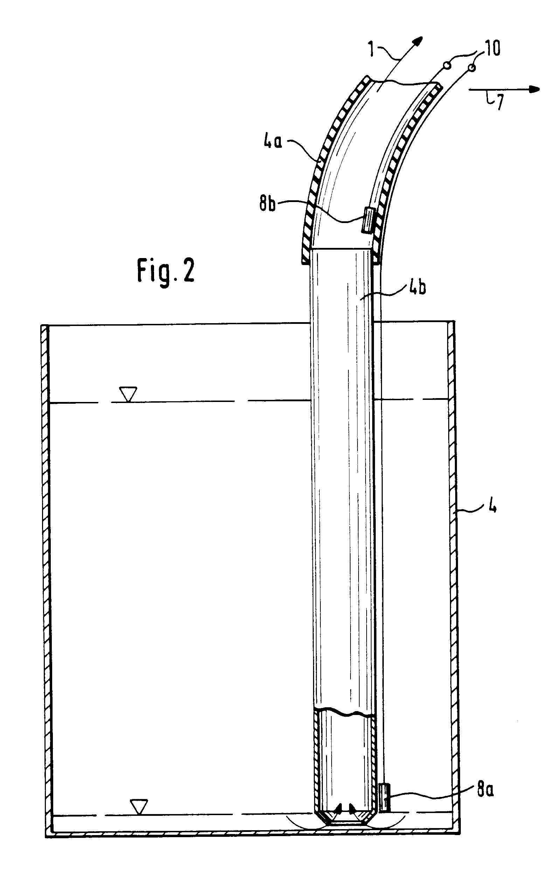

Nach einer vorteilhaften Ausgestaltung der Erfindung, die in Fig. 2 dargestellt ist, werden die Elektroden der Leitfähigkeitszellen in den Konzentratansaugleitungen so integriert, daß sie gleichzeitig als Füllstandssensoren für die Konzentratbehälter dienen können.

Die Fig. 2 zeigt den Konzentratbehälter 4 der Fig. 1 in vergrößerter schematischer Darstellung. Der Konzentratbehälter ist mittels des Zuführschlauches 4a an die Mischeinrichtung 1 angeschlossen. In das Konzentrat taucht das Ansaugrohr 4b ein, das aus einem Kunststoffmaterial hergestellt ist und das im unteren Bereich, angedeutet durch die Pfeile, die Ansaugöffnung besitzt. Oberhalb dieser Ansaugöffnungen ist an der Außenseite des Kunststoffrohres die erste Elektrode 8a der Leitfähigkeitszelle angebracht. Die zweite Elektrode 8b befindet sich im Zuführschlauch 4a, der nicht in die Konzentratlösung eintaucht.

Wird im Betrieb Konzentrat in das Innere des Ansaugrohres 4b angesaugt, so stellt sich an den Klemmen 10 ein Meßsignal für die Leitfähigkeit ein, sobald die Konzentratflüssigkeitssäule die zweite Elektrode 8b erreicht. Sinkt der Füllstand im Behälter unter das Niveau der ersten Elektrode 8a, so wird dieses Signal unterbrochen und es kann ein Alarmsignal erzeugt werden, bevor der Konzentralbehälter leer ist.

Derartige Füllstandsüberwachungen im Konzentratbehälter sind an sich bekannt; im bekannten Fall werden kontaktlose Nährungsschalter für die Erfassung des Füllstandes zusätzlich vorgesehen. Im Fall der Erfindung ist jedoch die gleichzeitige Verwendung der Elektroden 8a, b zur Leitfähigkeitsmessung und zur Füllstandsüberwachung wesentlich.

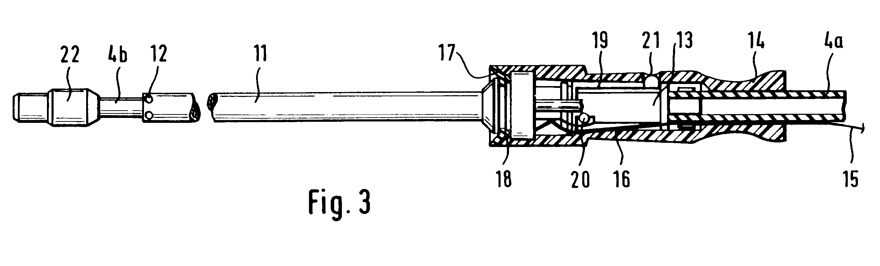

Die Fig. 3 zeigt eine teilweise geschnittene Darstellung eines kompletten Ansaugrohres, die im Unterschied zur Ansaugrohrleitung gemäß Fig.2 keine Leitfähigkeitszelle als solche aufweist, sondern bei der die Leitfähigkeit über ein elektrisch leitendes Metallrohr 11 und dessen radiale Ausgangsöffnungen 12 in der Überwachungsschaltung 7 registriert und angezeigt wird und die gleichzeitig als Füllstandsmesser dienen. Entsprechend dieser Ausführungsform geht der Zuführschlauch 4a an seinem vorderen Ende in ein Zwischenklemmstück 13 über, welches wiederum mit dem Ausgangsrohr 4b verbunden ist. Diese Übergangsstelle ist von einem Griffstück 14 umgeben, das aus einem Kunststoff besteht. Über nahezu den gesamten Bereich des Ansaugrohres 4b ist dieses von einem elektrisch leitenden Metallrohr 11 umhüllt, das an seinem hinteren Ende ebenfalls mit dem Zwischenklemmstück 13 elektrisch isoliert verbunden ist. Das elektrisch leitende Metallrohr 11 dient als Elektrode und ist mit einer im Zuführschlauch 4a befindlichen Kontaktleitung 15 über eine im Griffstück 14 angeordnete Kontaktfeder 16 in Verbindung. Das Griffstück 14 ist gegenüber dem Metallrohr 11 mittels einer Dichtscheibe 17 konzentratmitteldicht abgedichtet, damit keine Konzentratflüssigkeit in den Innenraum des Griffstückes 14 gelangen kann. Diese Dichtscheibe 17 ist durch einen Distanzring 18 festgelegt.

Weiterhin ist im Griffstück 14 ein Rastelement 19 vorgesehen, das über einen Stift 20 schwenkbar und in eine Grifföffnung 21 einrastbar ist. Dadurch kann bei Betätigung des Rastelementes 18 dieses zusammen mit dem elektrisch leitenden Metallrohr 11 aus der Halterung des Griffstückes 14 beliebig entfernt und ein anderes Ansaugrohr und/oder Metallrohr eingesetzt werden.

Am vorderen Ende des Metallrohres 11 im Bereich der Ansaugöffnungen 12, die O-ringförmig ausgebildet sein können und eine Strömungsverbindung vom Außenraum zum Innenraum des Ansaugrohres 4b gewährleisten, erstreckt sich ein Teil des Ansaugrohres 4b, an dem eine Codierbuchse 22 angeordnet ist, die das vordere Ende des Ansaugrohres 4b verschließt. Diese dient dazu, in ein im Konzentratbehälter angebrachtes entsprechendes Gegenstück einzugreifen, um eine Verwechslung der Konzentratbehälter mit dem entsprechenden Zuführschlauch auszuschließen. Dazu weist jede Codierbuchse eine andere Form oder einen anderen Querschnitt, beispielsweise kreis- oder rechteckigförmig auf.

In der Überwachungsschaltung 7 ist eine entsprechende Leitfähigkeit registriert. Die Kontaktleitung 15 und das Metallrohr 11 sind stromführend. Sinkt nun der Konzentratspiegel unterhalb der Ansaugöffnungen 12, besteht keine Leitfähigkeit mehr zwischen dem Konzentrat im Flüssigkeitsbehälter und dem Metallrohr 11, so daß das Nichtvorhandensein der Leitfähigkeit über die Kontaktleitung 15 an die Überwachungsschaltung 7 gemeldet wird, die ein entsprechendes Alarmsignal erzeugt bevor der Konzentratbehälter leer ist. Damit dient die elektrische Leitfähigkeit gleichzeitig der Füllstandsüberwachung, da die Ansaugöffnungen 12 am vorderen Ende des Metallrohres 11 die Funktion einer Leitfähigkeitszelle übernehmen.

## Patentansprüche

1. Vorrichtung für die Hämodialyse mit einer Mischeinrichtung (1), die Dialysierflüssigkeit für einen Dialysator (2) durch Mischung mindestens eines über eine Ansaugleitung (4a, b; 5a, b) aus einem Behälter zugeführten Dialysekonzentrates mit aufbereitetem Wasser herstellt, und mit einem vom Mischsystem unabhängigen, auf der Überwachung der Leitfähigkeit der Dialysierflüssigkeit beruhenden Schutzsystem (6, 7), das verhindert, daß Dialysierflüssigkeit mit einer für den Patienten gefährlicher Zusammensetzung zum Dialysator (2) gelangt, dadurch gekennzeichnet, daß in der Ansaugleitung (4a, b; 5a, b) eine zusätzliche Leitfähigkeitszelle (8; 9) angeordnet ist, deren Ausgangssignal auf eine Überwachungsstufe des Schutzsystems (7) geschaltet ist, an der die Soll-Leitfähigkeit bei richtigem Anschluß des Dialyse-Konzentratbehälters voreingestellt ist und die ein Alarm- oder Steuersignal abgibt, wenn die gemessene Leitfähigkeit aufgrund eines falschen Anschlusses des Dialyse-Konzentratbehälters von dem voreingestellten Wert abweicht.

2. Vorrichtung nach Anspruch 1 mit zwei Dialyse-Konzentratbehältern, dadurch gekennzeichnet, daß die Überwachungsstufe (7) derart ausgebildet ist, daß sie auf das Verhältnis der Ausgangssignale der zugehörigen Leitfähigkeitszellen (8, 9) anspricht.

3. Vorrichtung nach Anspruch 1 oder 2, dadurch gekennzeichnet, daß die Leitfähigkeitszellen (8, 9) derart in die Ansaugleitung (4a, b; 5a, b) integriert sind, daß sie gleichzeitig als Füllstandssensoren für den Dialyse-Konzentratbehälter dienen.

4. Vorrichtung nach Anspruch 3 mit einer Ansaugleitung, die aus einem nicht in das Konzentrat eintauchenden Zuführschlauch (4a, 5a) und einem in das Konzentrat eintauchenden Ansaugrohr (4b, 5b) besteht, dadurch gekennzeichnet, daß das Ansaugrohr (4b; 5b) aus einem Kunststoffmaterial besteht und an der Außenseite oberhalb der Ansaugöffnung die erste Elektrode (8a) der Leitfähigkeitszelle trägt, deren andere Elektrode (8b) im Innern des Zuführschlauchs (4a) angeordnet ist (Figur 2).

5. Vorrichtung nach Anspruch 3 mit einer Ansaugleitung (4a, 4b), die aus einem nicht in das Konzentrat eintauchenden Zuführschlauch (4a) und einem in das Konzentrat eintauchenden Ansaugrohr (4b) besteht, dadurch gekennzeichnet, daß das Ansaugrohr(4b) ein elektrisch leitendes Rohr (11) aufweist, das als Elektrode dient und über eine Leitung (15) mit der Überwachungsschaltung (7) verbunden ist.

6. Vorrichtung nach Anspruch 5, dadurch gekennzeichnet, daß das Ansaugrohr (4b) an seinem vorderen Ende zusätzlich mit einer Codierbuchse (22) versehen ist, die in ein entsprechendes Gegenstück im Konzentratbehälter greift.

7. Vorrichtung nach Anspruch 5 und/oder 6, dadurch gekennzeichnet,daß das Ansaugrohr (4b) lösbar über eine Kontaktfeder (16) mit der Leitung (15) und über Verrastelemente (20, 21) mit einem Griffstück (13) verbunden ist.

8. Vorrichtung nach mindestens einem der Ansprüche 5 bis 7, dadurch gekennzeichnet, daß das vordere Ende des Ansaugrohres (4b) von der Codierbuchse (22) verschlossen ist und im Bereich dieses Endes radiale Öffnungen (12) vorgesehen sind, die eine Strömungsverbindung vom Außenraum zum Innenraum des Ansaugrohres (4b) gewährleisten.

## Claims

1. Apparatus for hemodialysis comprising a mixing means (1) which prepares dialysis solution for a dialyzer (2) by mixing at least one dialysis concentrate supplied via an intake conduct (4a, b; 5a, b) from a container with prepared water, and a protective system (6, 7) which is independent of the mixing system, is based on the monitoring of the conductivity of the dialysis solution and prevents dialysis solution with a compositon dangerous to the patient reaching the dialyzer (2), characterized in that in the intake conduit (4a, b; 5a, b) an additional conductivity cell (8; 9) is disposed whose output signal is applied to a monitoring stage of the protective system (7) and at which (cell) the desired conductivity with correct connection of the dialysis concentrate container is preset and which (cell) emits an alarm or control signal when the measured conductivity due to an incorrect connection of the dialysis concentrate container deviates from the preset value.

2. Apparatus according to claim 1 comprising two dialysis concentrate containers, characterized in that the monitoring stage (7) is constructed in such a manner that it responds to the ratio of the output signals of the associated conductivity cells (8,9).

3. Apparatus according to claim 1 or 2, characterized in that the conductivity cells (8, 9) are integrated into the intake conduit (4a, b; 5a, b) in such a manner that they serve simultaneously as filling level sensors for the dialysis concentrate container.

4. Apparatus according to claim 3 comprising an intake conduit which consists of a feed flexible tube (4a, 5a) not dipping into the concentrate and an intake tube (4b, 5b) dipping into the concentrate, characterized in that the intake tube (4b; 5b) consists of a plastic material and at the outer side above the intake opening carries the first electrode (8a) of the conductivity cell, the other electrode (8b) of which is arranged in the interior of the feed flexible tube (4a) (Figure 2).

5. Apparatus according to claim 3 comprising an intake conduit (4a, 4b) which consists of a feed flexible tube (4a) not dipping into the concentrate and an intake tube (4b) dipping into the concentrate, characterized in that the intake tube (4b) comprises an electrically conductive tube (11) which serves as electrode and is connected via a line (15) to the monitoring Circuit (7).

6. Apparatus according to claim 5, characterized in that the intake tube (4b) is additionally provided at its front end with a coding jack (22) which engages into a corresponding counter member in the concentrate container.

7. Apparatus according to claim 5 and/or 6, characterized in that the intake tube (4b) is connected detachably via a contact spring (16) to the line (15) and via detent elements (20, 21) to a grip member (13).

8. Apparatus according to at least one of claims 5 to 7, characterized in that the front end of the intake tube (4b) is sealed by the coding jack (22) and in the region of said end radial openings (12) are provided which ensure a flow connection from the outer space to the inner space of the intake tube (4b).

## Revendications

1. Dispositif d'hémodialyse comportant un dispositif de mélange (1) qui prépare le liquide de dialyse pour un dialyseur (2), en mélangeant au moins un concentré de dialyse, provenant d'un réservoir, par un conduit d'aspiration (4a, b ; 5a, b), avec de l'eau traitée, et comportant un système de protection (6, 7), indépendant du système de mélange, reposant sur le contrôle de la conductibilité du liquide de dialyse, qui empêche que le liquide de dialyse parvienne au dialyseur (2) avec une composition dangereuse pour le patient, caractérisé en ce que dans le conduit d'aspiration (4a, b ; 5a, b) est montée une cellule de conductibilité (8 ; 9) supplémentaire dont le signal de sortie est envoyé à un étage de contrôle du système de protection (7) lequel est préréglé sur la conductibilité de consigne, lorsque le réservoir du concentré de dialyse est correctement raccordé et qui délivre un signal d'alarme ou de commande lorsque la conductibilité mesurée diffère de la valeur présélectionnée, à la suite d'un branchement incorrect du réservoir de concentré de dialyse.

2. Dispositif selon la revendication 1 comportant deux réservoirs de concentré de dialyse, caractérisé en ce que l'étage de contrôle (7) est conçu de manière à réagir au rapport entre les signaux de sortie des cellules de conductibilité (8, 9) correspondantes.

3. Dispositif selon la revendication 1 ou 2, caractérisé en ce que les cellules de conductibilité (8, 9) sont intégrées dans le conduit d'aspiration (4a, b ; 5a, b) de manière à servir simultanément de capteurs de niveau pour le réservoir de concentré de dialyse.

4. Dispositif selon la revendication 3, comportant un conduit d'aspiration qui est constitué d'un tuyau d'alimentation (4a, 5a), non immergé dans le concentré et d'un tube d'aspiration (4b, 5b) immergé dans le concentré, caractérisé en ce que le tube d'aspiration (4b ; 5b) est réalisé dans une matière plastique et porte, sur le côté extérieur, au-dessus de l'ouverture d'aspiration, la première électrode (8a) de la cellule de conductibilité dont l'autre electrode (8b) est montee a l'interieur du tuyau d'alimentation (4a) (figure 2).

5. Dispositif selon la revendication 3, comportant un conduit d'aspiration (4a, 4b) qui est constitué d'un tuyau d'alimentation (4a), non immergé dans le concentré et d'un tube d'aspiration (4b) immergé dans le concentré, caractérisé en ce que le tube d'aspiration (4b) comporte un tube (11) électriquement conducteur qui sert d'électrode et qui est relié au circuit de contrôle (7), par une ligne (15).

6. Dispositif selon la revendication 5, caractérisé en ce que le tube d'aspiration (4b) est pourvu en plus, à son extrémité avant, d'un connecteur de codage (22) qui s'engage dans une contre-pièce correspondante du réservoir de concentré.

7. Dispositif selon la revendication 5 et/ou 6, caractérisé en ce que le tube d'aspiration (4b) est assemblé de manière amovible avec la ligne (15), par un contact de ressort (16) et avec une pièce formant poignée (13), par des éléments d'accrochage (20, 21).

8. Dispositif selon l'une au moins des revendications 5 à 7, caractérisé en ce que l'extrémité avant du tube d'aspiration (4b) est fermée par le connecteur de codage (22) et en ce qu'il est prévu, dans la zone de cette extrémité, des ouvertures (12) radiales qui garantissent une liaison d'écoulement, du volume extérieur au volume intérieur du tube d'aspiration (4b).
